# EUROPEAN PATENT APPLICATION

(11) **EP 0 710 488 A1**
(43) Date of publication of application: **08.05.1996**
(21) Application number: 95402466.7
(22) Date of filing: 03.11.1995
(51) Int. Cl.: A61M 16/00

(54) **One-way valve with bacterial filter**

(30) Priority: 04.11.1994 US 19940334058
(71) Applicant: Stockett, Rick L., Phoenix, Arizona 85022 (US)
(72) Inventor: Stockett, Rick L., Phoenix, Arizona 85022 (US)
(74) Representative: Pernez, Helga

(57) **Abstract**

A one-way valve with bacterial filter apparatus (12) having a valve and filter assembly (20) with a shield member (22) connected thereto being extended laterally to provide a flexible impervious material to provide protection from bacterial transmission. The valve and filter assembly (20) includes 1) a respirator bottom assembly (24);2) a flapper valve member (26) operable to act as a one-way valve; 3) a bacterial filter assembly (28) to prevent transmission of bacteria therethrough in either direction; and 4) a respirator top assembly (30) mounted on the respirator bottom assembly (24) and having the bacterial filter assembly (28) mounted therein. The valve and filter assembly (20) has a one-way valve therein which permits air to be transmitted therethrough into a patient receiving CPR (16) but not permitting air flow from the patient receiving CPR (16) to the person applying the CPR (14). A fluid flow channel directs air flow from the person applying CPR (14) to the patient receiving CPR (16) through the bacterial filter assembly (28). Afluid flow exit channel directs airflowfrom the patient receiving CPR (16) laterally of the valve and filter assembly (20) and the flapper valve member (26) is closed to seal off the fluid flow channel to the person administering CPR (14).

## Description

A patent search was not conducted on this invention however the applicant is aware of the following United States patents relating to valve structures:

| Patent No. | Invention | Inventors |
|---|---|---|
| 4, 449,525 | PULMONARY RESUSCITATOR | White et al |
| 4,520,811 | PULMONARY RESUSCITATOR | White et al |
| 4,886,057 | ASSISTED BREATHING INTERFACE DEVICE | Jerald L. Nave |

The two (2) White et al patents are substantially identical and having a movable flapper valve member to seal on an inclined tube end.

The Nave patent regarding the Assisted Breathing Interface Device, with the inventor being a co-inventor of the prior two (2) White et al patents, is an improvement in a means in which a valve member seats during opening and closing.

### PREFERRED EMBODIMENT OF THE INVENTION

In one preferred embodiment of this invention, a one-way valve with bacterial filter apparatus is operable to apply artificial resuscitation or cardiopulmonary resuscitation, hereinafter referred to as CPR, to revive a patient who has stopped breathing due to a heart attack, electrical shock drowning, and the like wherein a person's heart has ceased to operate. The one-way valve with bacterial filter apparatus includes means to prevent transmission of germs through bacteria from a patient to a person applying CPR and vice versa.

The one-way valve with bacterial filter apparatus includes a valve and filter assembly having a user shield member or protector connected thereto.

The user shield member or protector is preferably constructed of a flexible plastic, impervious material which extends outwardly from the valve and filter assembly and acts to protect against the transmission of germs or bacteria from the person administering CPR to the patient receiving subject CPR.

The valve and filter assembly includes 1) a respirator bottom assembly; 2) a flapper valve member mounted within the respirator bottom assembly; 3) a bacterial filter assembly; and 4) a respirator top assembly having the bacterial filter assembly mounted therein and connected to the respirator bottom assembly

The respirator bottom assembly includes a patient's inlet section integral with a valve support and connector section which, in turn, is integral with and upper support rim section. The patient's inlet section is provided with an inlet opening to be inserted into a mouth portion of a person receiving CPR.

The valve support and connector section includes an outlet valve section having a patient discharge opening or hole therein to receive and convey any air or fluid flow received from a patient receiving CPR on partial or complete revival thereof. The fluid flow is directed away from the valve and filter assembly and further shielded from the person applying the CPR by the user shield member.

The upper support rim section is provided with a valve sealing surface to receive a portion of the flapper valve member to remounted thereon.

The flapper valve member includes an outer peripheral anchor section having a sealing valve section pivotally connected thereto.

The sealing valve section has a sealing surface engagable with the outlet valve section to provide a seal over the patient discharge opening during a CPR operation or function. This moves the air or fluid flow from the person giving CPR to flow downwardly through the inlet opening in the patient's inlet section to the patient during the administration of CPR. This achieves a fluid flow channel through the valve and filter assembly only as the patient discharge opening is sealed.

The bacterial filter assembly includes a filter retainer member and a bacterial filter member. The filter retainer member is operable as a retaining means for holding the bacterial filter member securely within the respirator top assembly as will be explained.

The bacterial filter member has an outer peripheral connector section which is clamped by the filter retainer member against the respirator top assembly.

The bacterial filter member is made of a material which prevents any bacteria or germs from filtering therethrough but allows air particles to do so.

The respirator top assembly includes a user's inlet section which is integral with a filter support section which, in turn, is integral with an outer valve sealing and support section.

On assembly, the outer valve sealing and support section will be connected and sealed with the upper support rim section of the respirator bottom assembly while having the user shield member clamped therebetween.

The user's inlet section is provided with a main inlet opening which is crossed with filter support struts. The inlet opening is utilized by the person administering CPR for breathing air therethrough.

The filter support struts provide a support surface for receiving the bacterial filter member thereagainst to prevent its movement outwardly during a CPR operation. The filter support struts in combination with the flapper valve member assures that the bacterial filter member will not become dislodged if the patient receiving CPR should start breathing.

The valve sealing and support section includes a sealing support flange engagable with the flapper valve member to hold in an assembled condition and a connector support flange. The connector support flange includes a connect surface engagable with a c ntral portion of the user shield member. When in the assembled condition, an air-tight real is achieved on clamping of the user shield member between the respirator bottom assembly and the respirator top assembly.

The user shield member is constructed of a plastic or bacteria non-pervious material and preferably of a circular shape.

### OBJECTS OF THE INVENTION

One object of this invention is to provide a one-way valve with bacterial filter apparatus having a one-way valve member in combination with a bacterial filter member wherein a person giving CPR to a patient is protected by allowing inlet air of the person giving CPR to pass into a fluid flow channel and into a mouth of the patient. Then, upon the patient starting to breathe, the subject one-way valve prevents discharge from the patient's mouth from coming into contact with the mouth of the person administering CPR during the process of applying CPR.

Another object of this invention is to provide a one-way valve with bacterial filter apparatus having a bacterial filter assembly and a user's shield member mounted thereon which is extended substantially laterally to add protection to prevent any discharge from the patient receiving CPR from reaching the person administering CPR during a CPR operation.

One other object of this invention is to provide a one-way valve with bacterial filter apparatus which can be mounted in a compact pouch for ease of conveyance; is of relatively small size so that it can be carried in a person's pocket and readily available for use; and is provided with protection from transmitting of bacterial diseases during a CPR operation for both the patient and the person giving CPR.

A further object of this invention is to provide a one-way valve with bacterial filter apparatus having a bacterial filter member with filter retaining features to assure that the bacterial filter member will not be dislodged during usage nor allow any bacteria to pass therethrough or around the bacterial filter member.

One further object of this invention is to provide a one-way valve with bacterial filter apparatus utilizing a flapper valve member which is movable in one direction to a first position on applying CPR to allow inlet air to pass directly to the patient receiving CPR in a fluid flow channel and movable in an opposite direction to a second position to allow air and fluid flow from the patient to flow in a fluid flow exit channel away from the person administering CPR.

Still, one other object of this invention is to provide a one-way valve with bacterial filter apparatus which is economical to manufacture; providing protection against transmittal of any bacteria and accompanying diseases, such as AIDS, being transmitted between the patient and the person applying CPR; is compact in size so as to be readily carried in a person's pocket; is readily disposed after each usage thereof; sturdy in construction; and substantially maintenance free.

Various other objects, advantages, and features of the invention will become apparent to those skilled in the art from the following discussion, taken in conjunction with the accompanying drawings, in which:

### FIGURES OF THE INVENTION

- Fig. 1: is a perspective view of the one-way valve with bacterial filter apparatus of this invention illustrated as being utilized by a person to a patient in a CPR function;
- Fig. 2: is a perspective view thereof having portions broken away for clarity;
- Fig. 3: is an enlarged sectional view taken along line 3-3 in Fig. 1;
- Fig. 4: is a fragmentary sectional view taken along line 4-4 in Fig. 3;
- Fig. 5: is an exploded perspective view thereof having a portion of a user shield member broken away for clarity;
- Fig. 6: is an enlarged sectional view taken along line 6-6 in Fig. 3;
- Fig. 7: is an enlarged sectional view taken along line 7-7 in Fig. 3; and
- Figs. 8 and 9: are schematic diagrams illustrating the use and operation of the one-way valve with bacterial filter apparatus of this invention.

The following is a discussion and description of preferred specific embodiments of the one-way valve with bacterial filter apparatus of this invention, such being made with reference to the drawings, whereupon the same reference numerals are used to indicate the same or similar parts and/or structure. It is to be understood that such discussion and description is not to unduly limit the scope of the invention.

### DESCRIPTION OF THE INVENTION

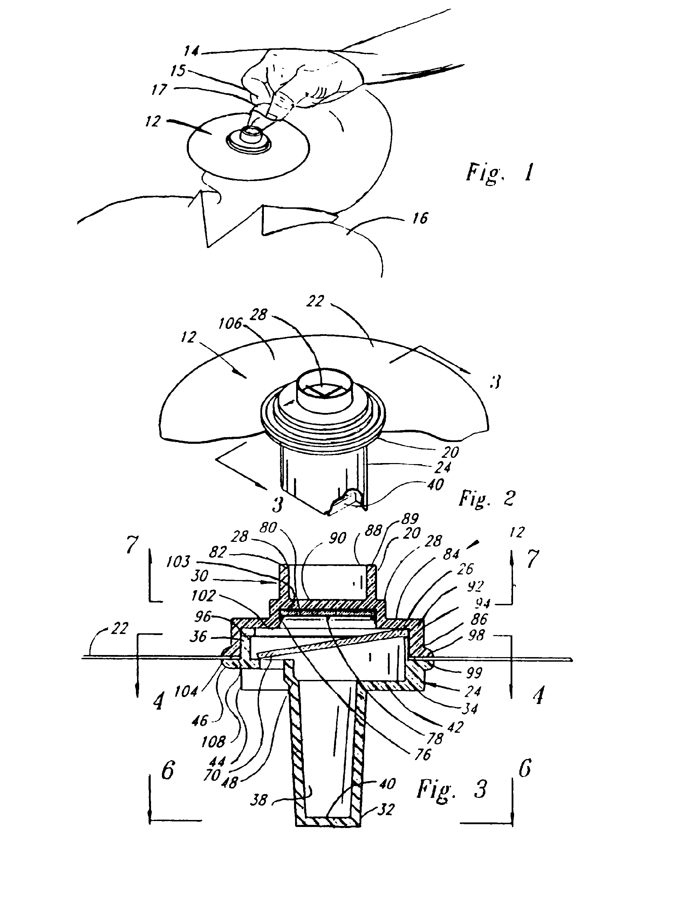

On referring to the drawings in detail and, in particular to Fig. 1, a one-way valve with bacterial filter apparatus of this invention, indicated generally at 12, is shown whereupon a patient 16 is in the process of receiving cardiopulmonary resuscitation (CPR) from a CPR person 14.

The one-way valve with bacterial filter apparatus 12 has a portion thereof inserted into an open mouth of the patient 16. The person 14 about to apply CPR is shown as having pinched a nose 17 of the patient 16 with its fingers 15 which is the first step before further proceeding with the CPR operation of breathing air therethrough into the patient 16.

As noted in Fig. 2, the one-way valve with bacterial filter apparatus 12 includes a valve and filter assembly 20 having attached about its periphery, at a central area, a shield member or protector 22 with the purpose thereof to be described in detail.

The valve and filter assembly 20 includes 1) a respirator bottom assembly 24; 2) a flapper valve member 26 mounted on an upper portion of the respirator bottom assembly 24; 3) a bacterial filter assembly 28 to be mounted above the flapper valve member 26: and 4) a respirator top assembly 30 adapted to receive and support the bacterial filter assembly 26 therein and functions to clamp the flapper valve member 26 in position. The user and patient snield member or protector 22 is to be clamped between the respirator bottom assembly 24 and the respirator top assembly 30 as will be explained.

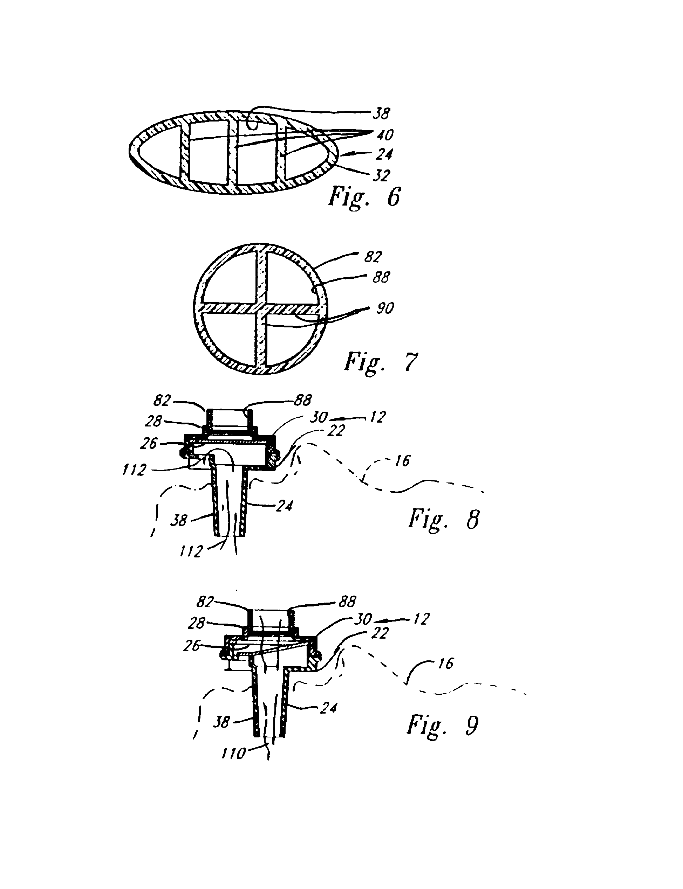

As best noted in Fig. 3, the respirator bottom assembly ?4 includes a patient's inlet section 32 of oval shape in transverse cross section (Fig. 6) and tapered downwardly which is integral with a valve support and connector section 34 which, in turn, is integral with an upper support rim section 36.

The patient inlet section 32 is of the oval and tapered shape so as to be readily inserted into the mouth of the patient 16 during a CPR operation and defines an inlet opening 38. A plurality of parallel, spaced support struts 40 across the inlet opening 38 adds rigidity plus keeps the inlet opening 38 open so that it will not be closed or deformed by actions of the patient 16 (such as clamping of teeth) during the CPR operation.

The valve support and connector section 34 includes a bottom wall 42 which is integral with a circular side wall 44 which is connected to 1) an outwardly extended support flange 46; and 2) an upper outlet valve section 48.

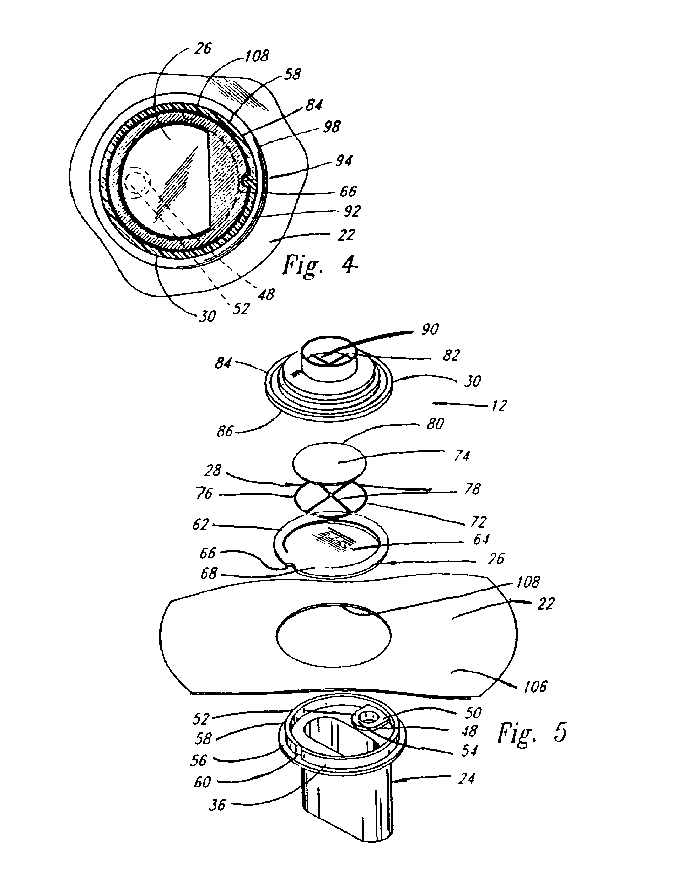

As noted in Fig. 5, the outlet valve section 48 includes a valve housing 50 having a central patient discharge opening or hole 52 therein which receives fluid flow to be discharged therethrough in a fluid flow exit channel during a CPR operation as will be explained. The valve housing 50 has an upper valve seating surface 54 operable to receive the flapper valve member 26 thereagainst in a sealing manner.

As best noted in Fig. 5, the upper support rim section 36 includes a side wall portion 56 having an upper valve sealing or clamping surface 58.

The ride wall portion 56 is provided with an alignment and locking notch 60 of semi-circular shape for the purposes of alignment and assembly. The alignment and locking notch 60 is positioned diametrically opposite of the patient discharge hole 52 to allow proper assembling procedures with the flapper valve member 26 as will be explained.

As noted in Fig. 5, the flapper valve member 26 is provided with an outer peripheral anchor section 62 having a movable sealing valve section 64 connected thereto. The peripheral anchor section 62 is provided with an alignment notch 66 of semi-circular shape to be utilized in assembly procedures.

The sealing valve section 64 is provided with a connector portion 68 interconnected to the peripheral anchor section 62 and having an inner lower sealing surface 70 which is operable to be selectively engagable with the patient discharge opening 52 in the outer valve section 48 of the valve support and connector section 34 as will be explained (See Fig. 9.).

As shown in Fig. 5, the bacterial filter assembly 28 includes a filter retainer member 72 and a bacterial filter member 74. The filter retainer member 72 has an outer ring section 76 of circular shape and having extended thereacross a pair of interconnected support struts 78.

The outer ring section 76 and interconnected support struts 78 are operable to engage and retain the bacterial filter member 74 in the assembled condition (Fig. 3).

The bacterial filter member 74 includes a main body section 30 operable to be engaged by the filter retainer member 72 in the assembled condition. The bacterial filter member 74 is constructed of a material that allows air flow therethrough but not the passage of germs and bacteria.

It has been found that a bacterial filter member 74 being manufactured by 3M Corporation entitled "6100 Filtrete Filter" is satisfactory being a bacterial and a viral filter.

As best shown in Fig. 3, the respirator top assembly 30 includes a user's inlet section 82 which is integral with a valve contact section 84 which, in turn, is integral with a valve sealing and support section 86. The user's inlet section 82 is provided with a central inlet opening 88 defined within an inlet side wall 89 and having filter support struts 90 extended thereacross to provide support to and prevent movement of the bacterial filter member 74 in the assembled condition.

The valve contact section 84 includes a side wall portion 92 and a retainer protrusion portion 94.

The valve sealing and support section 86 includes a sealing support flange 96 which is integral with a connector support flange 98. The sealing support flange 96 has a sealing surface 102 of circular shape operable to engage and seal with the peripheral anchor section 62 of the flapper valve member 26.

The connector support flange 98 has a connector surface 104 and having a plurality of spaced pointed projections or nubs 99 to aid in a sealing and holding function when clamping the user shield member 22 between the respirator bottom assembly 24 and the respirator top assembly 30. Concentric grooves can replace the nubs 99 to achieve the same purpose and functions. The projections 99 prevent unintentional movement of the user shield member 22 from its interconnection to the valve and filter assembly 20.

The shield member 22 is shown as being of circular shape and may extend several inches outwardly from the center of the valve and filter assembly 20. The shield member 22 is constructed of an impervious material, such as plastic or the like, and will not allow any fluid flow, germs, or bacteria to pass therethrough or the connection thereof to the valve and filter assembly 20.

The shield member 22 is a sheet member 106 having a central mounting hole 108 operable for connection to the valve and filter assembly 20.

### USE AND OPERATION OF THE INVENTION

An assembly of the one-way valve with bacterial filter apparatus 12 is readily understood when viewing the exploded perspective view of Fig. 5.

First, the respirator top assembly 30 can be assembled by placing the bacterial filter member 74 within the valve contact section 84. The bacterial filter member 74 is placed firmly against the filter support struts 90 in the user's inlet section 82.

Next, the filter retainer member 72 can be easily snapped within the valve contact section 84 and against the bacterial filter member 74. As noted, an inner surface of the valve contact section 84 is provided with an inwardly extended, circular contact rim or protrusion 103 which is slightly less in diameter than the outer periphery of the filter retainer member 72. This allows the filter retainer member 72 to be snapped therein and retained by the contact rim or protrusion 103.

In the next step, the shield member or protector 22 can be placed about the respirator bottom assembly 24 with the mounting hole 108 placed about the upper support rim section 36 of the respirator bottom assembly 24 as shown in Fig. 3.

Proceeding, the flapper valve member 26 is mounted on the upper valve sealing surface 58 of the upper support rim section 36 with the alignment notch 66 on the flapper vlave member 26 aligned with the alignment and locking notch 60 in the upper support rim section 36 of the respirator top assembly 30.

In n a final assembly step, the respirator top assembly 30 is mounted on the respirator bottom assembly 24 to assume the condition as shown in Fig. 3.

The retainer protrusion portion 94 on the valve contact section 84 acts as an alignment guide as positioned within the alignment notch 66 and the alignment and locking notch 60 as noted in Fig. 4.

By interlocking of the alignment notch 66, the alignment and locking notch 60, and the retainer protrusion portion 94, this assures proper assembly and operation of the flapper valve member 26 with the connector portion 68 diametrically opposite the patient discharge opening 52.

On use of the one-way valve with bacterial filter apparatus 12 in administrating a CPR function and on referring to Fig. 1, the CPR person 14 would first clear the air passageway of the patient 16 to receive CPR by known medical procedures. The patient inlet section 32 of the one-way valve and bacterial filter apparatus 12 would then be inserted into an open mouth of the patient 16 by the CPR person 14.

Next, as known in a CPR operation, the CPR person 14 would use its fingers 15 to close the nose 17 of the patient 16 so that any air to be administered through the valve and filter assembly 20 will be contained in the air passageway of the patient 16. This adds air to the lungs of the patient 16 which is then exhaled by forceful impacts to the lungs of the patient 16. Next, air is supplied to the lungs of the patient 16 and this two stage cycle is repeated.

This administration of CPR is shown in Fig. 9 whereupon the injected air from the CPR person 14 would move through the respirator top assembly 30 and move the flapper valve member 26 downwardly. The air from the CPR person 14 would travel about the outer edges of the sealing valve section 64 of the flapper valve member 26 in a fluid flow channel through the valve and filter assembly 20 as noted by arrows 110.

Concurrently, the sealing valve section 64 of the flapper valve member 26 would contact the valve seating surface 54 of the outlet valve section 48 so as to engage and seal with the patient discharge opening 52 to prevent fluid flow therethrough.

The shield member 22 provides further protection to the CPR person 14 from receiving any bacterial discharge from the patient 16 and vice versa on revival of the patient 16.

Upon the patient 16 proceeding to a condition of successful resuscitation, there may be some coughing and similar activity, including breathing by the patient 16. At this time, contaminated discharge, such as air and other matter, may flow upwardly into the valve and filter assembly 20 as noted by arrows 112 in Fig. 8.

At this time, the air pressure from the patient 16 would move the flapper valve member 26 upwardly and allow the air flow and discharge matter of the patient 16 to move as shown by the arrow 112 which will move outwardly through the patient discharge opening 52 in a fluid flow exit channel. At this time, the shield member 22 will offer protection to the CPR person 14 from possible contaminated discharge from the patient 16.

Further, in both of the operations of the one-way valve with bacterial filter apparatus 12 as shown in Figs. 8 and 9, it is noted that the bacterial filter assembly 28 provides a further protection as it does not allow germs, bacteria, and other similar material from passing therethrough thus providing safety to both the CPR person 14 and the patient 16 from transmitting communicable diseases such as AIDS, tuberculosis, and the like therebetween.

The one-way valve with bacterial filter apparatus is compact in construction; easy to convey; economical to manufacture; provided with many built in safety features including a bacterial filter member; easy tok use and does not require one skilled in the art to use during a CPR operation; and substantially maintenance free.

While the invention has been described in conjunction with preferred specific embodiments thereof, it will be understood that this description is intended to illustrate and not to limit the scope of the invention, which is defined by the following claims:

## Claims

1. A one-way valve with bacterial filter apparatus operable to be used when applying CPR to a patient, comprising:
a) a valve and filter assembly includes a fluid flow channel therethrough, and having a bacterial filter member and a flapper valve member, both extended transversely of said fluid flow channel;
b) said valve and filter assembly includes a fluid flow exit channel operable to receive fluid flow from the patient therethrough;
c) said flapper valve member moved into engagement with said fluid flow exit channel on fluid flow through said fluid flow channel to the patient; and
d) said flapper valve member moved out of engagement with said fluid flow exit channel when fluid flow from the patient is present in a portion of said fluid flow channel and exits in said fluid flow exit channel.

2. A one-way valve with bacterial filter apparatus as described in Claim 1, including:
a) a shield member connected to said valve and filter assembly and extended laterally outwardly therefrom constructed of a bacterial impervious material adapted to protect the person applying CPR from contamination from the patient receiving CPR.

3. A one-way valve with bacterial filter apparatus as described in Claim 1, including:
a) a bacterial filter assembly mounted transversely of said fluid flow channel having a filter retainer member to anchor said bacterial filter member; and
b) said bacterial filter member constructed of a material to prevent the flow of bacteria in any direction while allowing air flow therethrough.

4. A one-way valve with bacterial filter apparatus as described in Claim 1 wherein:
a) said valve and filter assembly having a respirator top assembly secured to a respirator bottom assembly;
b) said respirator top assembly includes an air inlet section having an inlet opening and filter support struts extended transversely thereof:
c) said respirator bottom assembly includes a patient's inlet section having an inlet opening with support struts extended transversely thereof;
d) said valve and filter assembly includes a filter retainer member extended across said fluid flow channel and said bacterial filter member having one side positioned against said filter support struts and another side positioned against said filter retainer member therethrough; and
e) said bacterial filter member is rigidly held in position by said filter support struts and said filter retainer member while allowing air flow without bacteria therethrough.

5. A one-way valve with bacterial filter assembly as described in Claim 4, wherein:
a) said respirator bottom assembly having said patient's inlet section of oval shape and tapered outwardly to said support struts for ease of insertion and receiving into a mouth portion of the patient having CPR applied thereto.

6. A one-way valve with bacterial filter apparatus operable to be inserted into a mouth of a person to receive CPR, comprising:
a) a valve and filter assembly having a fluid flow channel extended therethrough and a fluid flow exit channel in communication with said fluid flow channel; and
b) said valve and filter assembly includes a valve member 1) operable in one position to seal said fluid flow exit channel and permit fluid flow in one direction through said fluid flow channel: and 2) operable in another position to restrict fluid flow in an opposite direction within a portion of said fluid flow channel and discharge the fluid flow through said fluid flow exit channel.

7. A one-way valve with bacterial filter apparatus as described in Claim 6, including:
a) a shield member connected to and laterally extended from said valve and filter assembly

8. A one-way valve with bacterial filter apparatus as described in Claim 6, wherein:
a) said valve and filter assembly includes a bacterial filter assembly having a bacterial filter member extended and sealed across said fluid flow channel so that any fluid will flow in either direction transversely of said bacterial filter member but not permitting bacteria and virus to move transversely of said bacterial filter member.

9. A one-way valve with bacterial filter apparatus as described in Claim 7, wherein:
a) said shield member being electronically sealed with a peripheral area of said valve and filter assembly and constructed of an impervious material to provide a shield against transmission of infectious bacteria between the patient and the person applying CPR.

10. A one-way valve with bacterial filter apparatus used to apply CPR, comprising:
a) a valve and filter assembly including 1) a respirator bottom assembly having a patient's inlet section to be inserted into a mouth of a person receiving CPR; 2) a respirator top assembly connected to said respirator bottom assembly and having a user's inlet section to be placed into a mouth of a person applying CPR; 3) a fluid flow channel extended from said user's inlet section to said patient's inlet section; 4) a valve member mounted between said respirator bottom assembly and said respirator top assembly; and 5) a bacterial filter assembly mounted transversely of said fluid flow channel to prevent flow of bacteria material therethrough;
b) said respirator bottom assembly having a fluid flow exit channel having a patient discharge opening which is in communication with said fluid flow channel; and
c) said valve member operable 1) in one position to permit fluid flow in one direction through said fluid flow channel and block subject fluid flow through said patient discharge opening; and 2) in another position to permit fluid flow in an opposite direction through said patient's inlet section, said patient discharge opening in said fluid flow exit channel and prevent fluid flow into said user's inlet section.

11. A one-way valve with bacterial filter apparatus as described in Claim 10, including:
a) a shield member mounted between said respirator bottom assembly and said respirator top assembly and extended substantially laterally therefrom and constructed of an impervious material to assist in preventing infectious bacteria being transmitted from the patient to the person applying CPR.

12. A one-way valve with bacterial filter apparatus as described in Claim 10, wherein:
a) said respirator top assembly having a retainer protrusion portion;
b) said respirator bottom assembly having an alignment and locking notch operable to receive said retainer protrusion portion in an assembled condition;
c) said alignment and locking notch positioned opposite said patient discharge opening;
d) said valve member having an alignment notch therein positioned opposite of said patient discharge opening in the assembled condition; and
e) said alignment and locking notch, said retainer protrusion portion, and said alignment notch are all positioned at adjacent interlocked relationships when assembled to assure that said valve member acts in a proper manner for sealing said patient discharge opening during a user's CPR operation on a patient.

13. A one-way valve with bacterial filter apparatus as described in Claim 10, wherein:
a) said respirator bottom assembly having a laterally extended flange portion with a sealing surface thereon;
b) said respirator top assembly having a laterally extended flange section; and
c) a shield member mounted between said upper flange section and said lower flange section in a clamped relationship to provide an impervious seal therebetween.

14. A one-way valve with bacterial filter apparatus as described in Claim 13, wherein:
a) said flange section having a plurality of circumferentially spaced projections engaged with said shield member in the assembled condition to prevent lateral movement thereof and retain a positive sealing feature to prevent fluid flow between said shield member and said valve and filter assembly.

15. A one-way valve with bacterial filter apparatus as described in Claim 13, wherein:
a) said shield member is electronically welded to said flange portion and said flange section to provide additional safety features of achieving a quality, impervious seal therebetween.

16. A one-way valve with bacterial filter apparatus as described in Claim 13, wherein:
a) said flange section having a plurality of circumferentially spaced ridges engaged with said shield member in the assembled condition to prevent lateral movement thereof and retain a positive sealing feature to prevent fluid flow between said shield member and said valve and filter assembly.
